# EUROPEAN PATENT APPLICATION

(11) **EP 0 597 681 A1**
(43) Date of publication of application: **18.05.1994**
(21) Application number: 93308959.1
(22) Date of filing: 10.11.1993
(51) Int. Cl.: C12N 9/76, C12N 15/57

(54) **Expression vectors for the bovine trypsin and trypsinogen and host cells transformed therewith**

(30) Priority: 13.11.1992 US 977703
(71) Applicant: ELI LILLY AND COMPANY, Indianapolis, Indiana 46285 (US)
(72) Inventor: Greaney, Michael Gerard, Indianapolis, Indiana 46220 (US); Rosteck, Paul Robert, Jr., Indianapolis, Indiana 46237 (US)
(74) Representative: Hudson, Christopher Mark

(57) **Abstract**

DNA sequences encoding bovine trypsin and bovine trypsinogen are provided as are recombinant DNA vectors comprising these sequences. Host cells transformed with the trypsin and trypsinogen expression vectors are disclosed in context of means for producing bovine trypsin and bovine trypsinogen.

## Description

Trypsin is a protease which cleaves to the carboxyl side of lysine and argenine residues. Trypsin is produced in the form of a precursor or zymogen molecule called trypsinogen. Trypsinogen is converted to trypsin by the action of enteropeptidase.

The substrate specificity of trypsin provides a useful enzyme for conversion of biosynthetically produced molecules to preferred molecules. An example is the conversion of proinsulin to insulin via trypsin mediated removal of the connecting peptide. Trypsin is commercially available and is produced primarily by isolation from the pituitary glands of a variety of species. Bovine and porcine pancreases are particularly common sources of trypsin. Purification procedures utilized to purify trypsin for later use in bioconversion processes aim to remove undesirable copurifying proteases from the desired trypsin product.

Notwithstanding much effort at purification, various lots of trypsin contain variable amounts of contaminating proteases. Chymotrypsin is frequently present in minimal amounts in trypsin production lots. The presence of even a minor amount of a contaminating protease results in undesirable cleavage of various products when only the trypsin mediated cleavage is desired. Conversion of proinsulin to insulin via the action of trypsin is thus complicated by contaminants of other proteases. The present invention solves the problem of contaminating protease contaminationn by providing recombinant DNA expression systems for the biosynthetic production of bovine trypsin and trypsinogen. Thus,the present invention represents a significant advance in the art of typsin and trypsinogen production thereby greatly facilitating bio-conversion of precursor molecules.

The present invention discloses and claims DNA sequences which encode bovine trypsin and trypsinogen. Expression vectors useful for producing trypsin and trypsinogen are also disclosed and claimed as are host cells transformed with these expression vectors. The expression vectors and host cells of the present invention provide a convenienct source for trypsin and trypsinogen molecules, free of contaminating proteases which disrupt biosynthetic conversation processes.

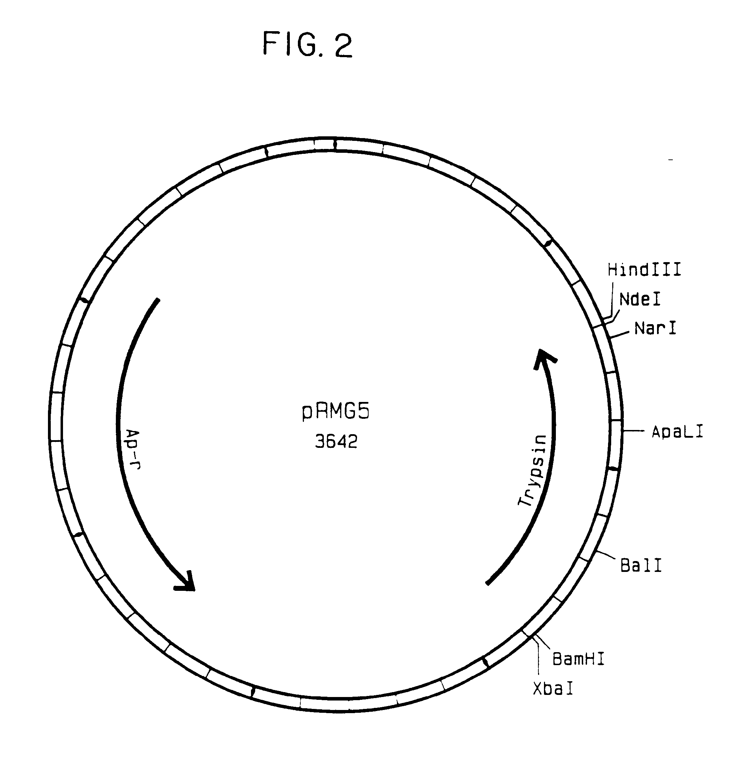

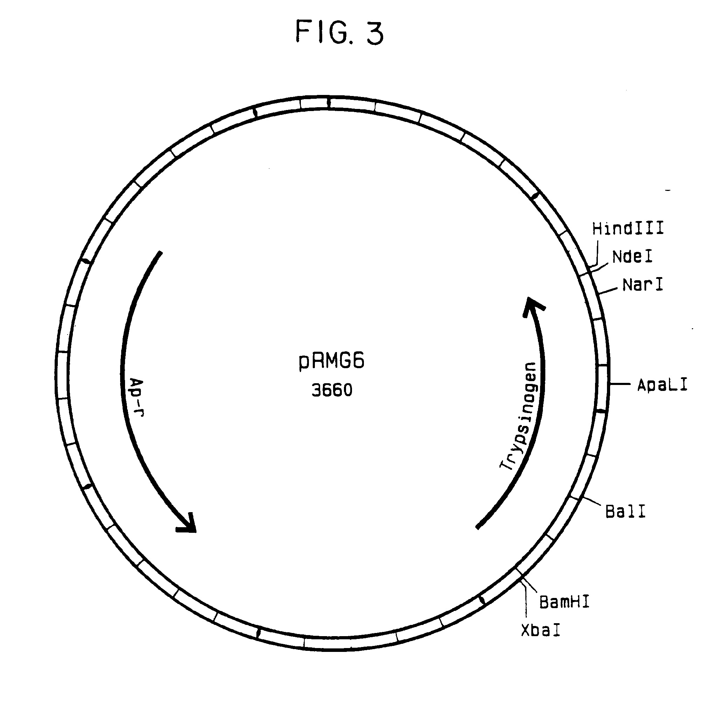

A series of figures are provided to further understanding of the invention. Figure 1 is a restriction site and function map of plasmid pRMG4. Figure 2 is a restriction site and function map of plasmid pRMG5. Figure 3 is a restriction site and function map of plasmid pRMG6. Figure 4 is a restriction site and function map of plasmid pRMG7. Figure 5 is a restriction site and function map of plsmid pHKY390.

The ability to produce trypsin either by direct expression or by production of the zymogen, trypsinogen affords flexibility in the isolation, purification and folding of trypsin by allowing the initial steps of trypsin production to be performed on an enzymatically inactive form.

The expression vectors provided by the instant invention were prepared by replacing the kanamycin phosphotransferase coding region of plasmid pHKY390 with chemically synthesized double-stranded DNA encoding trypsin or trypsinogen. Plasmid pHKY390 was deposited with the Northern Regional Research Laboratory (N.R.R.L.), Peoria, IL USA on January 17, 1992, where it is available under the accession number NRRL B-18885. Plasmid pHKY390 was deposited in the E. coli host strain RV308.

The chemically synthesized genes encoding trypsin and trypsinogen were prepared on an Applied Biosystems DNA synthesizer using (3-cyanoethyl phosphoramidite chemistry. A series of 20 oligonucleotides was synthesized as described in Example 1. The appropriate oligonucleotides were then annealed and ligated to generate double stranded DNA molecules encoding bovine trypsin and bovine trypsinogen. The double stranded DNA sequence which was prepared to encode bovine trypsin is provided below as Formula 1. The amino acid sequence encoded by the corresponding DNA is provided below the oligonucleotide sequence. Sequence I.D. 21, which is provided in a later section of this disclosure, corresponds to the sense strand of the sequence provided in Formula I. Sequence I.D. 22 corresponds to the amino acid sequence of Formula 1. The oligonucleotide sequences, which flank the coding sequence, are designated by lower case letters and the stop codon,TAG is designated as END in the amino acid sequence provided below the oligonucleotide sequence of Formula 1.

### Formula I

The double stranded sequence encoding bovine trypsin is provided to add detail to the single stranded format required in the Sequence Identification section of this disclosure. Restriction endonuclease recognition sites are provided above the sequence as appropriate; the amino acid encoded by each codon is presented below the DNA sequence; and the nucleotides forming the flanking regions of the coding region are provided to illustrate via restriction endonuclease recognition sites and linkers the mannerwhereby the coding sequence was inserted into the expression vectors.

The DNA sequence synthesized to comprise a bovine trypsinogen encoding region is provided below as Formula II. The format is similar to that provided above for the region encoding bovine trypsin(Formula I). Sequence I.D. 23, which is provided in a later section of this disclosure, corresponds to the coding sequence of Formula II while Sequence I.D. 24 provides the amino acid sequence encoded thereby. The oligonucleotide sequences, which flank the coding sequence, are designated by lower case letters and the stop codon,TAG is designated as END in the amino acid sequence provided below the oligonucleotide sequence of Formula II.

### Formula II

The gene for bovine trypsin was prepared by assembling subsets of the oligonucleotides described in Example 1 into three separate cassettes prior to combining the three cassettes to form the full length bovine trypsin encoding gene. Oligonucleotides BT1-6 were annealed and inserted into the commercially available vector, pBluescript SK+ (Stratagene). Oligonucleotide sequences BT7-12 were likewise annealed and inserted into a pBluescript SK+ cloning vector. The third cassette was generated upon ligation of oligonucleotides BT13-18 and insertion into a third pBluescript SK+ cloning vector. The three cassettes encoding portions of the bovine trypsin gene each have a Hind III termini and an Xbal termini. The bovine trypsin encoding sequence was synthesized as three separate components to minimize the chance for spontaneous mutations occurring within the sequence. The cloning vector comprising oligonucleotides BT1-6 is designated pRMG1. The cloning vector comprising oligonucleotides BT7-12 is designated plasmid pRMG2. The cloning vector comprising oligonucleotides BT13-18 is designated pRMG3. The three portions of the bovine trypsin encoding sequence were prepared by digesting plasmids pRMG1, pRMG2, and pRMG3with appropriate endonucleases followed by ligation of the fragments and insertion into an expression vector. The expression vector utilized in the construction of trypsin and trypsinogen expression vectors is designated plasmid pHKY390. Plasmid pHKY390 has been deposited in the Northern Regional Research Laboratory, Peoria, IL where it is publicly available under the accession number B-1885.

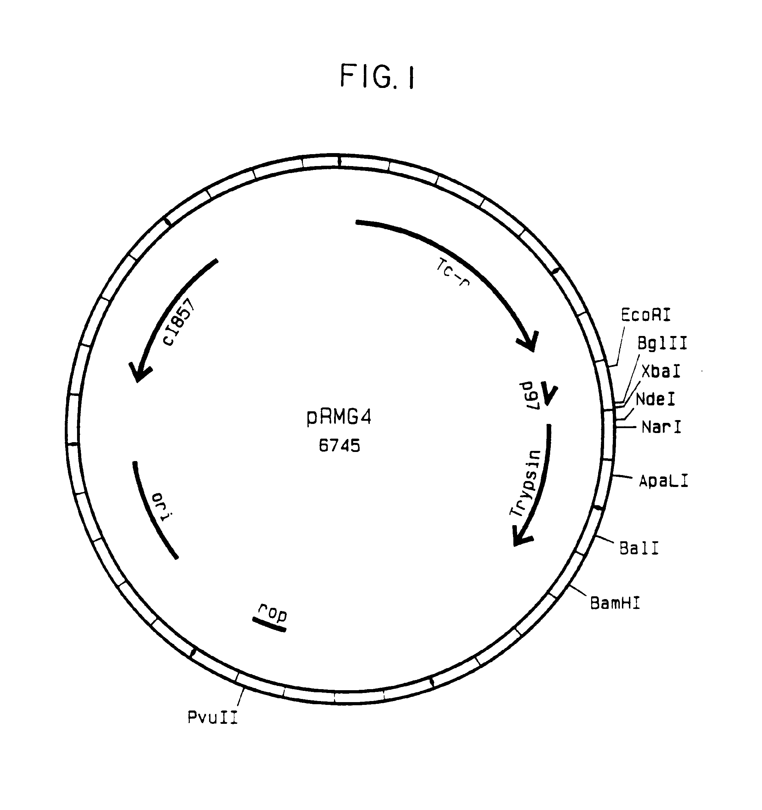

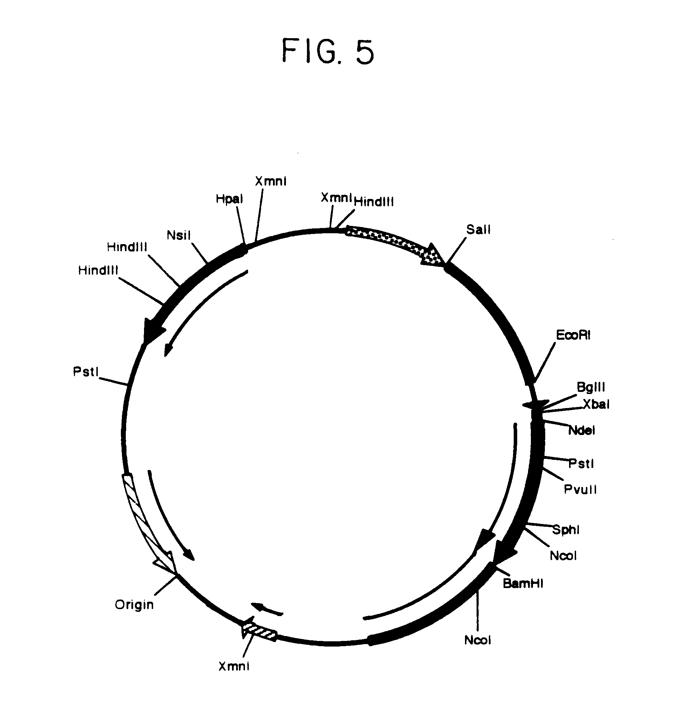

A restriction site and function map of plasmid pHKY390 is provided in Figure 5. Plasmid pHKY390 was originally used as a promoter probe wherein promoters were evaluated for their ability to cause transcription of the kanamycin phosphotransferase gene of plasmid pHKY390. Reference to Figure 5 reveals that an Ndel and BamHl site are conveniently located in plasmid pHKY390 for insertion of a sequence encoding a polypeptide product of interest . Plasmid pRMG4 was constructed by insertion of the trypsin encoding gene into the Ndel/BamHl digested plasmid pHKY390. The three fragments which upon ligation generate the trypsin encoding gene were prepared as described in Example 4. A restriction site and function map of plasmid pRMG4 is provided in Figure 1. Plasmid pRMG4 utilizes a modified lambda pL promoter, p97, to drive transcription of a two cistron message wherein the second cistron encodes bovine trypsin. Plasmid pRMG4 uses a tetracyline resistance gene as a selectable marker. The temperature sensitive lambda pL repressor, c1857, is utilized to provide regulatable transcription from the modified lambda promoter. The origin of replication utilized in plasmid pRMG4 was prepared originally from plasmid pBR322. Plasmid pRMG4 also utilizes a rop gene. The rop gene provides a vector copy number of approximately fifteen to twenty when utilized, as in the vectors of the present invention, with a pBR322-derived origin of replication.

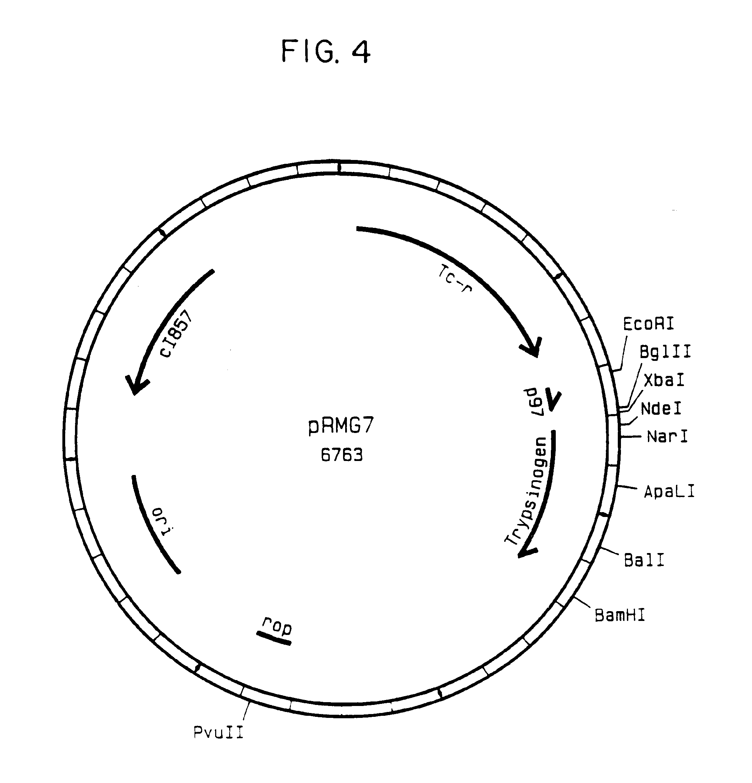

Plasmid pRMG7 is the preferred expression vector for bovine trypsinogen. Reference to Figures 1 and 4 and the examples indicates the high level of similarity between the preferred expression vectors for bovine trypsin and bovine trypsinogen. Accordingly the description of the elements in plasmid pRMG4 is likewise applicable to plasmid pRMG7.

A variety of E. coli host cells were utilized in the construction of the vectors and expression systems of the present invention. E. coli RV308 is available from the Northern Regional Research Laboratory, Peoria, IL (NRRL) under the accession number NRRL B-15624 E. coli MM294 is available from the American Tissue Culture Collection, Parklawn , MD (ATCC) under the accession number ATCC 31446. The inability of either of these strains to support expression of bovine trypsin or bovine trypsinogen from plasmids pRMG4 and pRMG7 respectively underscores the unpredictability, which remains in the art of molecular biology. The reason or reasons why such well recognized E. coli host strains were incapable of achieving expression of trypsin and trypsinogen remains unelucidated. Digestion of either the messenger RNA or the desired protein product could account for the failure to affect expression in these strains. E. coli L687, a lon- host cell, was eventually tried and this host cell strain proved to be competent for expression of bovine trypsin and bovine trypsinogen from pRMG4 and pRMG7 respectively. E. coli L687 was deposited in the NRRL where it is available under the accession number B-18884. Accordingly, E. coli L687 transformed with plasmids pRMG4 and pRMG7 comprise the respective best modes for producing bovine trypsinogen and bovine trypsin in prokaryotic cells. The media utilized in the fermentative production of the enzyme and zymogen of the present invention affect the overall production levels of the desired products. L-broth is the preferred media for such fermentation processes. The components of L-broth are 1 % (w/v) Bacto tryptone; 0.5% (w/v) Yeast extract; 0.5% (w/v) NaCI; and 0.1 % (w/v) dextrose at pH 7.0. L-agar is L-broth solidified with 1.5% (w/v) Bacto agar.

The expression products of plasmids pRMG4 and pRMG7 have been established by conventional biochemical methodologies to be bovine trypsin and trypsinogen respectively. The availability of trypsin, whether expressed directly or converted from its zymogen precursor, provides a signifigant advantage in biochemical conversion processes such as the removal of the connecting peptide of insulin. The source of enzyme devoid of contaminating proteases allows substantially greater flexibility in the production of important medicinal polypeptides such as insulin. The biosynthetic source of the enzyme also eliminates any concerns related to the use of enzymes prepared from animal sources in the production of molecules which will be administered to humans or animals.

The examples which follow are intended to further illustrate the present invention and are not to be interpreted as limiting on the scope thereof. While the examples and detailed description sections of the present invention are sufficient to guide anyone of ordinary skill in the art in the practice of the present invention, skilled artisans are also directed to Molecular Cloning A Laboratory Manual Second Edition, Sambrook,J., Fritsch, E. F., and Maniatis, T., Cold Spring Harbor Press 1989 and Current Protocols In Molecular Biology, Ausubel, F.M., Brent,R., Kingston,R.E., Moore, D. D., Seidman,J.G., Smith, J.A.,and Struhl, K.,Ed. Greene Publishing Associates and Wiley-Interscience 1989. The aforementioned resources provide an excellent technical supplement to any discourse in genetic engineering.

The examples provide sources for reagents, however it will be understood that numerous vendors market reagents of high quality for use in the protocols and procedures described below and the substitution of reagents or protocols is contemplated by the present invention and embraced in the scope thereof. All temperatures unless otherwise noted are expressed in degrees Centigrade. All percentages are on a weight per weight basis unless otherwise noted.

### Example 1

### Oligonucleotide synthesis and purification

The following oligonucleotides were synthesized on an Applied Biosystems (Foster City, CA) model 380B DNA synthesizer using beta-cyanoethyl phosphoramidite chemistry according to the manufacturer's instructions. The single stranded DNA segments were conventionally purified on 12% polyacrylamide-7M urea gels and resuspended in water.

BT1 , (Sequence I.D. 1)(Sequence Length: 77)

BT2 (Sequence I.D 2) (Sequence Length: 77)

BT3A (Sequence I.D. 3) (Sequence Length: 81)

BT4A (Sequence I.D. 4) (Sequence Length: 81)

BT5 (Sequence I.D. 5) (Sequence Length: 73)

BT6 Sequence I.D. 6) (Sequence Length: 73)

BT7 (Sequence I.D. 7) (Sequence Length: 84)

BT8 (Sequence I.D. 8) (Sequence Length: 84)

BT9 (Sequence I.D. 9) (Sequence Length: 93)

BT10 (Sequence I.D. 10) (Sequence Length: 93)

BT11 (Sequence I.D. 11) (Sequence Length: 88)

BT12 (Sequence I.D. 12)(Sequence Length: 88)

BT13 (Sequence I.D. 13) (Sequence Length: 77)

BT14 (Sequence I.D. 14) (Sequence Length: 77)

BT15 (Sequence I.D. 15) (Sequence Length: 76)

BT16 (Sequence I.D. 16) (Sequence Length: 76)

BT17 (Sequence I.D. 17) (Sequence Length: 74)

BT18 (Sequence I.D. 18)(Sequence Length: 74)

BT19 (Sequence I.D. 19) (Sequence Length: 45)

BT20 (Sequence I.D. 20) (Sequence length: 45)

### Example 2

### Construction of pRMG1

### A. Preparation of 231 base pair Hindlll-Xbal gene segment

Six µg of oligonucleotides BT2, BT3A, BT4A, and BT5 were individually phosphorylated in 20 µl reactions containing 50 mM Tris-HCI (pH 7.8), 10 mM MgC1₂, 5 mM dithiothreitol, 5% glycerol, 100 µM adenosine triphosphate, and 20 units T4 polynucleotide kinase (Boehringer Mannheim, Indianapolis, IN) at 37°C for 30 min. The kinase was thermally inactivated by heating at 70°C for 5 min.

Six µg of each of the above phosphorylated oligonucleotides was mixed with 6 µg (6µl) each of oligonucleotides BT1 and BT6, heated at 70° C for 5 min. and cooled to room temperature to allow the oligonucleotides to anneal. The annealed oligonucleotides were then treated with 30 units T4 DNA ligase (Boehringer Mannheim, Indianapolis, IN) in a 200 µl reaction containing 50 mM Tris-HCI (pH 7.8), 10 mM MgC1₂, 5 mM dithiothreitol, 5% glycerol,and 100 µM adenosine triphosphate for 1 hour at 20°C then 18 hours at 15°C.

The desired 231 base pair DNA fragment was conventionally purified on an 8% polyacrylamide gel and resuspended in water. Two µg of the purified DNA fragment was treated with 20 units of T4 polynucleotide kinase in a 20 µ1 reaction containing 50 mM Tris-HCI (pH 7.8), 10 mM MgCl₂, 5 mM dithiothreitol, 5% glycerol, and 100 µM adenosine triphosphate at 37°C for 30 min.

### B. Preparation of pBluescript SK+ vector

Twenty µg of plasmid pBluescript SK+ (Stratagene, LaJolla, CA) was digested to completion with 100 units Hindlll (Boehringer Mannheim, Indianapolis, IN) and 100 units Xbal (Boehringer Mannheim, Indianapolis, IN) in a 250 µl reaction containing 50 mM Tris-HCI (pH 8.0), 10 mM MgCl₂, 50 mM NaCI, and 100 µg/ml bovine serum albumin at 37°C for one hour. The enzymes were thermally inactivated by heating at 70°C for 10 min.

The 5' termini were dephosphorylated by treatment of the DNAwith 5 units (5 µl) calf intestinal alkaline phosphatase (Boehringer Mannheim, Indianapolis, IN) at 37°C for 30 min. The enzyme was thermally inactivated by heating at 70°C for 15 min. The solution was extracted with an equal volume of phenol equilibrated with 100 mM Tris-HCI (pH 8.0). The aqueous layer was recovered and DNAwas precipitated by the addition of 0.1 volume 3 M sodium acetate and 2.2 volumes of absolute ethanol. The DNAwas collected by centrifugation and resuspended in 300 µl water.

### C. Final construction of pRMG1

1.3 µg of the purified 231 base pair fragment prepared in Example 2A and 0.3 µg of the pBluescript vector DNA prepared in Example 1 B were ligated with 10 units of T4 DNA ligase in a 10 µl reaction containing 50 mM Tris-HCI (pH 7.8), 10 mM MgC1₂, 5 mM dithiothreitol, 5% glycerol,and 100 µM adenosine triphosphate at 20°C for 18 hours.

A portion of the ligation mixture was used to transform E. coli K12 MM294 cells. Transformants were selected on Lagar containing 50 µg/ml ampicillin. Ampicillin-resistant transformants containing the desired plasmid pRMG1 were identified following plasmid DNA purification by restriction enzyme site analysis and nucleotide sequencing.

### Example 3

### Construction of pRMG2

### A. Preparation of 265 base pair Hindlll-Xbal gene segment

Six µg of oligonucleotides BT8, BT9, BT10, and BT11 were individually phosphorylated in 20 µl reactions containing 50 mM Tris-HCI (pH 7.8), 10 mM MgC1₂, 5 mM dithiothreitol, 5% glycerol, 100 µM adenosine triphosphate, and 20 units T4 polynucleotide kinase (Boehringer Mannheim, Indianapolis, IN) at 37°C for 30 min. The kinase was thermally inactivated by heating at 70°C for 5 min.

Six µg of each of the above phosphorylated oligonucleotides was mixed with 6 µg (6µl) each of oligonucleotides BT7 and BT12, heated at 70° C for 5 min. and cooled to room temperature to allow the oligonucleotides to anneal. The annealed oligonucleotides were then treated with 30 units T4 DNA ligase (Boehringer Mannheim, Indianapolis, IN) in a 200 µl reaction containing 50 mM Tris-HCI (pH 7.8), 10 mM MgC1₂, 5 mM dithiothreitol, 5% glycerol, and 100 µM adenosine triphosphate for 1 hour at 20°C then 18 hours at 15°C.

The desired 265 base pair DNA fragment was conventionally purified on an 8% polyacrylamide gel and resuspended in water. Two µg of the purified DNA fragment was treated with 20 units of T4 polynucleotide kinase in a 20 µl reaction containing 50 mM Tris-HCI (pH 7.8), 10 mM MgC1₂, 5 mM dithiothreitol, 5% glycerol, and 100 µM adenosine triphosphate at 37°C for 30 min.

### B. Final construction of pRMG2

1.3 µg of the purified 265 base pair fragment prepared in Example 2A and 0.3 µg of the pBluescript vector DNA prepared in Example 1 B were ligated with 10 units of T4 DNA ligase in a 10 µl reaction containing 50 mM Tris-HCI (pH 7.8), 10 mM MgC1₂, 5 mM dithiothreitol, 5% glycerol, and 100 µM adenosine triphosphate at 20°C for 18 hours.

A portion of the ligation mixture was used to transform Ecoli K12 MM294 cells. Transformants were selected on Lagar containing 50 µg/ml ampicillin. Ampicillin-resistant transformants containing the desired plasmid pRMG2 were identified following plasmid DNA purification by restriction enzyme site analysis and nucleotide sequencing.

### Example 4

### Construction of pRMG3

### A. Preparation of 227 base pair Hindlll-Xbal gene segment

Six µg of oligonucleotides BT14, BT15, BT16, and BT17 were individually phosphorylated in 20 µl reactions containing 50 mM Tris-HCI (pH 7.8), 10 mM MgC1₂, 5 mM dithiothreitol, 5% glycerol, 100 µM adenosine triphosphate, and 20 units T4 polynucleotide kinase (Boehringer Mannheim, Indianapolis, IN) at 37°C for 30 min. The kinase was thermally inactivated by heating at 70°C for 5 min.

Six µg of each of the above phosphorylated oligonucleotides was mixed with 6 µg (6µl) each of oligonucleotides BT13 and BT18; heated at 70° C for 5 min. and cooled to room temperature to allow the oligonucleotides to anneal. The annealed oligonucleotides were then treated with 30 units T4 DNA ligase (Boehringer Mannheim, Indianapolis, IN) in a 200 µl reaction containing 50 mM Tris-HCI (pH 7.8), 10 mM MgCl₂, 5 mM dithiothreitol, 5% glycerol, and 100 µM adenosine triphosphate for 1 hour at 20°C then 18 hours at 15°C.

The desired 227 base pair DNA fragment was conventionally purified on an 8% polyacrylamide gel and resuspended in water. Two µg of the purified DNA fragment was treated with 20 units of T4 polynucleotide kinase in a 20 µl reaction containing 50 mM Tris-HCI (pH 7.8), 10 mM MgCl₂, 5 mM dithiothreitol, 5% glycerol, and 100 µM adenosine triphosphate at 37°C for 30 min.

### B. Final construction of pRMG3

1.3 µg of the purified 227 base pair fragment prepared in Example 3A and 0.3 µg of the pBluescript vector DNA prepared in Example 2B were ligated with 10 units of T4 DNA ligase in a 10 µl reaction containing 50 mM Tris-HCI (pH 7.8), 10 mM MgCl2, 5 mM dithiothreitol, 5% glycerol, and 100 µM adenosine triphosphate at 20°C for 18 hours.

A portion of the ligation mixture was used to transform E. coli K12 MM294 cells. Transformants were selected on Lagar containing 50 µg/ml ampicillin. Ampicillin-resistant transformants containing the desired plasmid pRMG3 were identified following plasmid DNA purification by restriction enzyme site analysis and nucleotide sequencing.

### Example 5

### Construction of pRMG4

### A. Preparation of the 218 Base Pair ApaLl-Ndel Restriction Fragment of pRMG1

Thirty µg of plasmid pRMG1 was digested to completion with 120 units of ApaLl (New England Biolabs, Beverly MA) in a 600µl reaction containing 10 mM Tris-HCI (pH 7.5), 10mM MgCl₂,and 1 mM dithiothreitol, and 100 µg/ml bovine serum albumin at 37°C for two hours. The enzyme was thermally inactivated by heating at 70°C for 10 min. The DNAwas digested to completion with Ndel by supplementing the reaction with 50 mM Tris-Hcl (pH 7.5), 100 mM Nacl and 120 units Ndel (Boehringer Mannheim, Indianapolis, IN) in a 750 µl reaction and incubating at 37°C for two hours. The enzyme was thermally inactivated by heating at 70°C for 10 min. The DNA was recovered by ethanol precipitation as described in example 1 B and resuspended in water. The desired 218 base pair fragment was conventionally purified on a 1.5% agarose gel by electroelution onto DEAE cellulose paper and resuspended in water.

### B. Preparation of the 247 Base Pair Mscl-ApaL1 Restriction Fragment of pRMG2

Thirty µg of pRMG2 was digested to completion with 75 units (25µl) Mscl (an isoschizomer of Ball, New England Biolabs, Beverly, MA) and 120 units (12µl) ApaLl in a 750 µl reaction containing 10 mM Tris-HCI (pH 7.5), 10mM MgCl₂, 1 mM dithiothreitol, and 100 µg/ml bovine serum albumin at 37°C for two hours. The enzymes were thermally inactivated by heating at 70°C for 10 min. The DNAwas recovered by ethanol precipitation as described in example 1 B and resuspended in water. The desired 247 base pair fragment was conventionally purified on a 1.5% agarose gel by electroelution onto DEAE cellulose paper and resuspended in water.

### C. Preparation of the 211 Base Pair Mscl-BamHl Restriction Fragment of pRMG3

Thirty µg pf pRMG3 was digested to completion with 75 units (25 µl) Mscl in a 600 µl reaction containing 50 mM potassium acetate, 20 mM Tris-acetate (pH 7.9), 10 mM magnesium acetate, 1 mM dithiothreitol, and 100 µg/ml bovine serum albumin at 37°C for 2 hours. Tris-acetate is Trizma@ acetate (Tris[hydroxymethyl]aminomethane acetate) and is available from Sigma Chemical Co., St. Louis, MO 63187. The enzyme was thermally inactivated at 70°C for 10 min. The DNA was digested to completion with BamHl by supplementing the reaction with 50 mM NaCl and 120 units of BamHl in a 750 µl reaction and incubating at 37°C for 2 hours. The enzyme was thermally inactivated by heating at 70°C for 10 min. The DNA was recovered by ethanol precipitation as described in example 1 B and resuspended in water. The desired 211 base pair fragment was conventionally purified on a 1.5% agarose gel by electroelution onto DEAE cellulose paper and resuspended in water.

### D. Preparation of pHKY390 expression vector

Twenty µg of plasmid pHKY390 was digested to completion with 240 units Ndel (Boehringer Mannheim, Indianapolis, IN) and 80 units BamHl (Boehringer Mannheim, Indianapolis, IN) in a 100 microliter reaction containing 50mM Tris-HCI (pH 8.0), 10mM MgCl₂, 100mM NaCi, and 100 µg/ml bovine serum albumin at 37°C for 1 hr. The enzymes were thermally inactivated by heating at 70°C for 10 min.

The 5' termini were dephosphorylated by treatment of the DNAwith 5 units (5 µl) calf intestinal alkaline phosphatase (Boehringer Mannheim, Indianapolis, IN) at 37°C for 30 min. The enzyme was thermally inactivated by heating at 70°C for 15 min. The solution was extracted with an equal volume of phenol equi- libarted with 100 mM Tris-HCI (pH 8.0). The aqueous layer was recovered and DNA was precipitated by the addition of 0.1 volume 3 M sodium acetate and 2.2 volumes of absolute ethanol. The DNAwas collected by centrifugation and resuspended in 300 µl water.

### E. Final construction of pRMG4

Two hundred ng of the purified 218 base pairfragment prepared in Example 5A, 200 ng of the purified 247 base pair fragment purified in Example 5B, 200 ng of the purified 211 base pair fragment purified in Example 5C, and 100 ng of the pHKY390 vector DNA prepared in Example 5D were ligated with 10 units of T4 DNA ligase (Boehringer Mannheim, Indianapolis IN) in a 20 µl reaction containing 50 mM Tris-HCI (pH 7.8), 10 mM MgC1₂, 5 mM dithiothreitol, 5% glycerol, and 100 µM adenosine triphosphate at 15°C for 15 hours.

A portion of the ligation mixture was used to transform E. coli K12 MM294 cells. Transformants were selected on L agar containing 10 µg/ml tetracycline. Tetracycline resistant transformants containing the desired plasmid pRMG4 were identified following plasmid DNA purification by restriction enzyme site analysis and nucleotide sequencing of the trypsin gene.

### Example 6

### Construction of pRMG5

### A. Preparation of the 225 base pair ApaLl-Hindlll restriction fragment of pRMG1

Twenty µg of pRMG1 was digested to completion with 80 units of ApaL1 (New England Biolabs, Beverly, MA) in a 100 µl reaction containing 10 mM Tris-HCI (pH 7.5), 10 mM MgC1₂, 1 mM dithiothreitol, and 100µg/ml bovine serum albumin at 37°C for 2 hours. The enzyme was thermally inactivated by heating at 70°C for 10 min. The DNAwas digested to completion with Hindlll by supplementing the reaction with 50 mM Tris-HCI (pH 7.5), 50 mM NaCI, and 80 units of Hindlll in a 125 µl reaction and incubating at 37°C for 2 hours. The enzyme was thermally inactivated by heating at 70°C for 10 min. The DNA was recovered by ethanol precipitation as described in Example 2B and resuspended in water. The desired 225 base pair fragment was conventionally purified on a 1.5% agarose gel by electroelution onto DEAE cellulose paper and resuspended in water.

### B. Preparation of pRMG3 vector

Thirty µg of pRMG3 was digested to completion with 75 units of Mscl ( an isoschizomer of Bal1, New England Biolabs, Beverly, MA) in a 600 µl reaction containing 50 mM potassium acetate, 20 mM Tris acetate, 10 mM magnesium acetate, 1 mM dithiothreitol, and 100 µg/ml bovine serum albumin at 37°C for 2 hours. The enzyme was thermally inactivated by heating at 70°C for 10 min. The DNA was digested to completion with Hindlll by supplementing the reaction with 50 mM Tris-HCI, 50 mM NaCi, and 120 units of Hindlll in a 750 µl reaction and incubating at 37°C for 2 hours. The enzyme was thermally inactivated at 70°C for 10 min.

The 5' termini were dephosphorylated and the DNAwas recovered by ethanol precipitation as described in Example 1 B and resuspended in water.

### C. Final construction of pRMG5

Two hundred ng of the purified 225 base pair fragment prepared in example 5A, 200 ng of the 247 base pair fragment prepared in Example 4B, and 50 ng of the pRMG3 vector DNA prepared in Example 6B were ligated with 10 units of T4 DNA ligase in a 40 µl reaction containing 50 mM Tris-Hcl (pH 7.8), 10 mM MgC1₂, 5 mM dithiothreitol, 5% glycerol, and 100 µM adenosine triphosphate at 15°C for 15 hours.

A portion of the ligation mixture was used to transform E. coli K12 MM294 cells. Transformants were selected on L agar containing 50 µg/ml ampicillin. Ampicillin resistant transformants containing the desired plasmid pRMG5 were identified following plasmid DNA purification by restriction enzyme site analysis and nucleotide sequencing of the trypsin gene.

### Example 7

### Construction of pRMG6

### A. Preparation of the 45 base pair Ndel-Narl segment

Seven µg of oligonucleotides BT19 and BT20 were individually phosphorylated in 20 µl reactions containing 50 mM Tris-HCI (pH7.8), 10 mM MgC1₂, 5 mM dithiothreitol, 5% glycerol, 100 µM adenosine triphosphate, and 20 units of T4 polynucleotide kinase (Boehringer Mannheim, Indianapolis, IN) at 37°C for 30 min. The kinase was thermally inactivated by heating at 70°C for 10 min.

The two 20 µl reactions were subsequently mixed, then heated to 70°C for 5 min. and cooled to room temperature to allow the BT19 and BT20 oligonucleotides to anneal.

### B. Preparation of pRMG5 vector

Twenty µg of pRMG5 was digested to completion with 40 units of Narl (Bethesda Research Laboratories, Gaithersburg, MD) in a 100 µl reaction containing 50 mM Tris-HCI (pH 8.0), 10 mM MgC1₂, and 100µg/ml bovine serum albumin at 37°C for 2 hours. The enzyme was thermally inactivated by heating at 70°C for 10 min. The DNA was digested to completion with Ndel by supplementing the reaction with 50 mM NaCl and 80 units of Ndel in a 125 µl reaction and incubating at 37°C for 2 hours. The enzyme was thermally inactivated by heating at 70°C for 10 min.

The 5' termini were dephosphorylated and the DNAwas recovered by ethanol precipitation as described in Example 1 B and resuspended in water.

### C. Final construction of pRMG6

Three hundred and fifty ng of the 45 base pair Narl-Ndel fragment prepared in Example 7A and 100 ng of the pRMG5 vector DNA prepared in Example 7B were ligated with 10 units of T4 DNA ligase in a 20 µl reaction containing 50 mM Tris-HCI (pH 7.8), 10 mM MgC1₂, 5 mM dithiothreitol, 5% glycerol, and 100 µM adenosine triphosphate at 15°C at 15 hours.

A portion of the ligation mixture was used to transform E. coli K12 MM294 cells. Transformants were selected on Lagar containing 50 µg/ml ampicillin. Ampicillin-resistant transformants containing the desired pRMG6 DNA were identified following plasmid DNA purification by restriction enzyme site analysis and nucleotide sequencing of the trypsinogen gene.

### Example 8

### Construction of pRMG7

### A. Preparation of the 695 base pair BamHI-Ndel trypsinogen gene

Twenty µg of plasmid pRMG6 was digested to completion with 36 units of BamHl (Boehringer Mannheim, Indianapolis, IN) and 20 units of Ndel (New England Biolabs, Beverly, MA) in a 40 µl reaction containing 50 mM Tris-HCI (pH 8.0), 10 mM MgC1₂, 100 mM NaCi, and 100 µg/ml bovine serum albumin at 37°C for 1 hour. The enzymes were thermally inactivated by heating at 70°C for 10 min. The DNA was recovered by ethanol precipitation as described in Example 2B and resuspended in water.

### B. Final construction of pRMG7

Three hundred and fifty ng of the restricted pRMG6 DNA prepared in Example 8A and 100 ng of the pHKY390 vector DNA prepared in Example 5D were ligated with 10 units of T4 DNAligase in a 25 µl reaction containing 50 mM Tris-HCI (pH 7.8), 10 mM MgCl₂, 5 mM dithiothreitol, 5% glycerol, and 100 µM adenosine triphosphate at 15°C for 15 hours.

A portion of the ligation mixture was used to transform E. coli K12 MM294 cells. Transformants were selected on L agar containing 10 µg/ml tetracycline. Tetracycline-resistant transformants containing the desired plasmid pRMG7 were identified following plasmid DNA purification by restriction enzyme site analysis and nucleotide sequencing of the trypsinogen gene.

### Example 9

### Construction of L693/pRMG4

### A. Transformation of L693 with pRMG4

The E. coli strain L693 was transformed with plasmid pRMG4 DNAfrom Example 4E. Transformants were selected on L agar containing 10µg/ml tetracycline. Tetracycline-resistant transformants containing the desired plasmid pRMG4 were identified by restriction enzyme site analysis and nucleotide sequencing of the trypsin gene.

### Example 10

### Construction of L687/pRMG7

### A. Transformation of L687 with pRMG7

The lon E. coli strain L687 was transformed with plasmid pRMG7 DNA from Example 8B. Transformants were selected on L agar containing 10µg/ml tetracycline. Tetracycline-resistant transformants containing the desired pRMG7 were identified by restriction enzyme site analysis and nucleotide sequencing of the trypsinogen gene.

### SEOUENCE LISTING

### (1) GENERAL INFORMATION:

(i) APPLICANT: ELI LILLY AND COMPANY
   (B) STREET: Lilly Corporate Center
   (C) CITY: Indianapolis
   (D) STATE: Indiana
   (E) COUNTRY: United States of America
   (F) ZIP: 46285
(ii) TITLE OF INVENTION: Expression Vectors for Bovine Trypsin and Trypsinogen and Host Cells Transformed Therewith
(iii) NUMBER OF SEQUENCES: 24
(iv) CORRESPONDENCE ADDRESS:
   (A) ADDRESSEE: C. M. Hudson
   (B) STREET: Erl Wood Manor
   (C) CITY: Windlesham
   (D) STATE: Surrey
   (E) COUNTRY: United Kingdom
   (F) ZIP: GU20 6PH
(v) COMPUTER READABLE FORM:
   (A) MEDIUM TYPE: Diskette, 3.50 inch, 1.0 Mb storage
   (B) COMPUTER: Macintosh
   (C) OPERATING SYSTEM: Macintosh
   (D) SOFTWARE: Microsoft Word

### (2) INFORMATION FOR SEQ ID NO: 1

(i) SEQUENCE CHARACTERISTICS:
   (A) LENGTH: 77 base pairs
   (B) TYPE: Nucleic acid
   (C) STRANDEDNESSS: single stranded
   (D) TOPOLOGY: linear
(ii) MOLECULE TYPE: DNA
(xi) SEQUENCE DESCRIPTION: SEQ ID NO: 1
   AGCTTCATAT GATCGTTGGC GGCTACACCT GTGGCGCCAA TACCGTCCCG    50
   TACCAGGTGT CCCTGAATTC TGGCTAC                             77

### (2) INFORMATION FOR SEQ ID NO: 2

(i) SEQUENCE CHARACTERISTICS:
(A) LENGTH: 77 base pairs
   (B) TYPE: Nucleic acid
   (C) STRANDEDNESSS: single stranded
   (D) TOPOLOGY: linear
(ii) MOLECULE TYPE: DNA
(xi) SEQUENCE DESCRIPTION: SEQ ID NO: 2

Sequence I.D 2

### (2) INFORMATION FOR SEQ ID NO: 3

(i) SEQUENCE CHARACTERISTICS:
(A) LENGTH: 81 base pairs
   (B) TYPE: Nucleic acid
   (C) STRANDEDNESSS: single stranded
   (D) TOPOLOGY: linear
(ii) MOLECULE TYPE: DNA
(xi) SEQUENCE DESCRIPTION: SEQ ID NO: 3

Sequence I.D. 3) (Sequence Length: 81)

### (2) INFORMATION FOR SEQ ID NO: 4

(i) SEQUENCE CHARACTERISTICS:
   (A) LENGTH: 81 base pairs
   (B) TYPE: Nucleic acid
   (C) STRANDEDNESSS: single stranded
   (D) TOPOLOGY: linear
(ii) MOLECULE TYPE: DNA
(xi) SEQUENCE DESCRIPTION: SEQ ID NO: 4

### Sequence I.D. 4

### (2) INFORMATION FOR SEQ ID NO: 5

(i) SEQUENCE CHARACTERISTICS:
   (A) LENGTH: 73 base pairs
   (B) TYPE: Nucleic acid
   (C) STRANDEDNESSS: single stranded
   (D) TOPOLOGY: linear
(ii) MOLECULE TYPE: DNA
(xi) SEQUENCE DESCRIPTION: SEQ ID NO: 5
   CTGGGCGAGG ATAACATCAA CGTCGTGGAG GGCAATGAGC AGTTCATCTC 50
   CGCATCCAAG TCCATCGTGC ACT 73

### (2) INFORMATION FOR SEQ ID NO: 6

(i) SEQUENCE CHARACTERISTICS:
   (A) LENGTH: 73 base pairs
   (B) TYPE: Nucleic acid
   (C) STRANDEDNESSS: double stranded
   (D) TOPOLOGY: linear
(ii) MOLECULE TYPE: DNA
(xi) SEQUENCE DESCRIPTION: SEQ ID NO: 6
   CTAGAGTGCA CGATGGACTT GGATGCGGAG ATGAACTGCT CATTGCCCTC 50
   CACGACGTTG ATGTTATCCT CGC 73

### (8) INFORMATION FOR SEQ ID NO: 7

(i) SEQUENCE CHARACTERISTICS:
   (A) LENGTH: 84 base pairs
   (B) TYPE: Nucleic acid
   (C) STRANDEDNESSS: single stranded
   (D) TOPOLOGY: linear
(ii) MOLECULE TYPE: DNA
(xi) SEQUENCE DESCRIPTION: SEQ ID NO: 7
   AGCTTCATCG TGCACCCGTC CTACAACTCC AACACTCTGA ACAATGACAT 50
   CATGCTGATC AAGCTCAAGT CCGCCGCATC CCTG 84

### (2) INFORMATION FOR SEQ ID NO: 8

(i) SEQUENCE CHARACTERISTICS:
   (A) LENGTH: 84 base pairs
   (B) TYPE: Nucleic acid
   (C) STRANDEDNESSS: single stranded
   (D) TOPOLOGY: linear
(ii) MOLECULE TYPE: DNA
(xi) SEQUENCE DESCRIPTION: SEQ ID NO: 8
   AGTTCAGGGA TGCGGCGGAC TTGAGCTTGA TCAGCATGAT GTCATTGTTC 50
   AGAGTGTTGG AGTTGTAGGA CGGGTGCACG ATGA 84

### (2) INFORMATION FOR SEQ ID NO: 9

(i) SEQUENCE CHARACTERISTICS:
   (A) LENGTH: 93 base pairs
   (B) TYPE: Nucleic Acid
   (C) STRANDEDNESSS: single stranded
   (D) TOPOLOGY: linear
(ii) MOLECULE TYPE: DNA
(xi) SEQUENCE DESCRIPTION: SEQ ID NO: 9
   AACTCCCGCG TGGCCTCCAT CTCTCTGCCG ACCTCCTGTG CCTCCGCCGG 50
   CACGCAGTGC CTCATCTCTG GCTGGGGCAA CACTAAGAGC TCT 93

### (2) INFORMATION FOR SEQ ID NO: 10

(i) SEQUENCE CHARACTERISTICS:
   (A) LENGTH: 93 base pairs
   (B) TYPE: Nucleic acid
   (C) STRANDEDNESSS: single stranded
   (D) TOPOLOGY: linear
(ii) MOLECULE TYPE: DNA
(xi) SEQUENCE DESCRIPTION: SEQ ID NO: 10
   TGCCAGAGCT CTTAGTGTTG CCCCAGCCAG AGATGAGGCA CTGCGTGCCG 50
   GCGGAGGCAC AGGAGGTCGG CAGAGAGATG GAGGCCACGC GGG 93

### (2) INFORMATION FOR SEQ ID NO: 11

(i) SEQUENCE CHARACTERISTICS:
   (A) LENGTH: 88 base pairs
   (B) TYPE: Nucleic acid
   (C) STRANDEDNESSS: single stranded
   (D) TOPOLOGY: linear
(ii) MOLECULE TYPE: DNA
(xi) SEQUENCE DESCRIPTION: SEQ ID NO: 11
   GGCACCTCCT ACCCAGACGT GCTGAAGTGC CTGAAGGCTC CTATCCTGAG 50
   CGATTCCTCC TGTAAGTCCG CCTACCCTGG CCAGATTT 88

### (2) INFORMATION FOR SEQ ID NO: 12

(i) SEQUENCE CHARACTERISTICS:
   (A) LENGTH: 88 base pairs
   (B) TYPE: Nucleic acid
   (C) STRANDEDNESSS: single stranded
   (D) TOPOLOGY: linear
(ii) MOLECULE TYPE: DNA
(xi) SEQUENCE DESCRIPTION: SEQ ID NO: 12
   CTAGAAATCT GGCCAGGGTA GGCGGACTTA CAGGAGGAAT CGCTCAGGAT 50
   AGGAGCCTTC AGGCACTTCA GCACGTCTGG GTAGGAGG 88

### (2) INFORMATION FOR SEQ ID NO: 13

(i) SEQUENCE CHARACTERISTICS:
   (A) LENGTH: 77 base pairs
   (B) TYPE: Nucleic acid
   (C) STRANDEDNESSS: single stranded
   (D) TOPOLOGY: linear
(ii) MOLECULE TYPE: DNA
(xi) SEQUENCE DESCRIPTION: SEQ ID NO: 13

### (2) INFORMATION FOR SEQ ID NO: 14

(i) SEQUENCE CHARACTERISTICS:
   (A) LENGTH: 77 base pairs
   (B) TYPE: Nucleic acid
   (C) STRANDEDNESSS: single stranded
   (D) TOPOLOGY: linear
(ii) MOLECULE TYPE: DNA
(xi) SEQUENCE DESCRIPTION: SEQ ID NO: 14
   CAGAATCACC CTGACAGGAA TCCTTGCCGC CCTCCAGGTA GCCGGCACAG 50
   AACATGTTGC TGGTAATCTG GCCAGGA 77

### (2) INFORMATION FOR SEQ ID NO: 15

(i) SEQUENCE CHARACTERISTICS:
   (A) LENGTH: 76 base pairs
   (_{B}) TYPE: Nucleic acid
   (C) STRANDEDNESSS: single stranded
   (D) TOPOLOGY: linear
(ii) MOLECULE TYPE: DNA
(xi) SEQUENCE DESCRIPTION: SEQ ID NO: 15
   TCTGGTGGCC CTGTGGTCTG CTCCGGCAAG CTCCAAGGCA TCGTCTCCTG 50
   GGGTTCCGGC TGTGCCCAGA AGAACA 76

### (2) INFORMATION FOR SEQ ID NO: 16

(i) SEQUENCE CHARACTERISTICS:
   (A) LENGTH: 76 base pairs
   (B) TYPE: Nucleic acid
   (C) STRANDEDNESSS: single stranded
   (D) TOPOLOGY: linear
(ii) MOLECULE TYPE: DNA
(xi) SEQUENCE DESCRIPTION: SEQ ID NO: 16
   GGCTTGTTCT TCTGGGCACA GCCGGAACCC CAGGAGACGA TGCCTTGGAG 50
   CTTGCCGGAG CAGACCACAG GGCCAC 76

### (2) INFORMATION FOR SEQ ID NO: 17

(i) SEQUENCE CHARACTERISTICS:
   (A) LENGTH: 74 base pairs
   (B) TYPE: Nucleic acid
   (C) STRANDEDNESSS: single stranded
   (D) TOPOLOGY: linear
(ii) MOLECULE TYPE: DNA
(xi) SEQUENCE DESCRIPTION: SEQ ID NO: 17
   AGCCTGGCGT CTACACCAAG GTCTGTAACT ATGTGTCCTG GATTAAGCAG 50
   ACCATAGCTT CCAATTAGGA TCCT 74

### (2) INFORMATION FOR SEQ ID NO: 18

i) SEQUENCE CHARACTERISTICS:
   (A) LENGTH: 74 base pairs
   (B) TYPE: Nucleic acid
   (C) STRANDEDNESSS: single stranded
   (D) TOPOLOGY: linear
(ii) MOLECULE TYPE: DNA
(xi) SEQUENCE DESCRIPTION: SEQ ID NO: 18
   CTAGAGGATC CTAATTGGAA GCTATGGTCT GCTTAATCCA GGACACATAG 50
   TTACAGACCT TGGTGTAGAC GCCA 74

### (2) INFORMATION FOR SEQ ID NO: 19

(i) SEQUENCE CHARACTERISTICS:
   (A) LENGTH: 45 base pairs
   (B) TYPE: Nucleic acid
   (C) STRANDEDNESSS: single stranded
   (D) TOPOLOGY: linear
(ii) MOLECULE TYPE: DNA
(xi) SEQUENCE DESCRIPTION: SEQ ID NO: 19
   TATGGTGGAT GATGATGATA AGATCGTTGG CGGCTACACC TGTGG 45

### (2) INFORMATION FOR SEQ ID NO: 20

(i) SEQUENCE CHARACTERISTICS:
   (A) LENGTH: 45 base pairs
   (B) TYPE: Nucleic acid
   (C) STRANDEDNESSS: single stranded
   (D) TOPOLOGY: linear
(ii) MOLECULE TYPE: DNA
(xi) SEQUENCE DESCRIPTION: SEQ ID NO: 20
   CGCCACAGGT GTAGCCGCCA ACGATCTTAT CATCATCATC CACCA 45

### (2) INFORMATION FOR SEQ ID NO: 21

(i) SEQUENCE CHARACTERISTICS:
   (A) LENGTH: 683 base pairs
   (B) TYPE: Nucleic acid
   (C) STRANDEDNESSS: double stranded
   (D) TOPOLOGY: linear
(ii) MOLECULE TYPE: DNA
(xi) SEQUENCE DESCRIPTION: SEQ ID NO: 21
   CAT ATG ATC GTT GGC GGC TAC ACC TGT GGC GCC AAT ACC GTC CCG    45
       Met Ile Val Gly Gly Tyr Thr Cys Gly Ala Asn Thr Val Pro    14
   TAC CAG GTG TCC CTG AAT TCT GGC TAC CAC TTC TGT GGT GGC TCC    90
   Tyr Gln Val Ser Leu Asn Ser Gly Tyr His Phe Cys Gly Gly Ser    29
   CTC ATC AAC TCC CAG TGG GTG GTA TCA GCG GCC CAC TGC TAC AAG   135
   Leu Ile Asn Ser Gln Trp Val Val Ser Ala Ala His Cys Tyr Lys    44
   TCC GGC ATC CAG GTG CGT CTG GGC GAG GAT AAC ATC AAC GTC GTG   180
   Ser Gly Ile Gln Val Arg Leu Gly Glu Asp Asn Ile Asn Val Val    59
   GAG GGC AAT GAG CAG TTC ATC TCC GCA TCC AAG TCC ATC GTG CAC   225
   Glu Gly Asn Glu Gln Phe Ile Ser Ala Ser Lys Ser Ile Val His    74
   CCG TCC TAC AAC TCC AAC ACT CTG AAC AAT GAC ATC ATG CTG ATC   270
   Pro Ser Tyr Asn Ser Asn Thr Leu Asn Asn Asp Ile Met Leu Ile    89
   AAG CTC AAG TCC GCC GCA TCC CTG AAC TCC CGC GTG GCC TCC ATC   315
   Lys Leu Lys Ser Ala Ala Ser Leu Asn Ser Arg Val Ala Ser Ile   104
   TCT CTG CCG ACC TCC TGT GCC TCC GCC GGC ACG CAG TGC CTC ATC   360
   Ser Leu Pro Thr Ser Cys Ala Ser Ala Gly Thr Gln Cys Leu Ile   119
   TCT GGC TGG GGC AAC ACT AAG AGC TCT GGC ACC TCC TAC CCA GAC   405
   Ser Gly Trp Gly Asn Thr Lys Ser Ser Gly Thr Ser Tyr Pro Asp   134
   GTG CTG AAG TGC CTG AAG GCT CCT ATC CTG AGC GAT TCC TCC TGT   450
   Val Leu Lys Cys Leu Lys Ala Pro Ile Leu Ser Asp Ser Ser Cys   149
   AAG TCC GCC TAC CCT GGC CAG ATT ACC AGC AAC ATG TTC TGT GCC   495
   Lys Ser Ala Tyr Pro Gly Gln Ile Thr Ser Asn Met Phe Cys Ala   164
   GGC TAC CTG GAG GGC GGC AAG GAT TCC TGT CAG GGT GAT TCT GGT   540
   Gly Tyr Leu Glu Gly Gly Lys Asp Ser Cys Gln Gly Asp Ser Gly   179
   GGC CCT GTG GTC TGC TCC GGC AAG CTC CAA GGC ATC GTC TCC TGG   585
   Gly Pro Val Val Cys Ser Gly Lys Leu Gln Gly Ile Val Ser Trp   194
   GGT TCC GGC TGT GCC CAG AAG AAC AAG CCT GGC GTC TAC ACC AAG  630
   Gly Ser Gly Cys Ala Gln Lys Asn Lys Pro Gly Val Tyr Thr Lys  209
   GTC TGT AAC TAT GTG TCC TGG ATT AAG CAG ACC ATA GCT TCC AAT  675
   Val Cys Asn Tyr Val Ser Trp Ile Lys Gln Thr Ile Ala Ser Asn  224
   taggatcc                                                     683

### (2) INFORMATION FOR SEQ ID NO: 22

(i) SEQUENCE CHARACTERISTICS:
   (A) LENGTH: 224
   (B) TYPE: protein
   (C) STRANDEDNESSS: single stranded
   (D) TOPOLOGY: linear
(ii) MOLECULE TYPE: Protein
(xi) SEQUENCE DESCRIPTION: SEQ ID NO: 22
   Met Ile Val Gly Gly Tyr Thr Cys Gly Ala Asn Thr Val Pro Tyr  15
   Gln Val Ser Leu Asn Ser Gly Tyr His Phe Cys Gly Gly Ser Leu  30
   Ile Asn Ser Gln Trp Val Val Ser Ala Ala His Cys Tyr Lys Ser  45
   Gly Ile Gln Val Arg Leu Gly Glu Asp Asn Ile Asn Val Val Glu  60
   Gly Asn Glu Gln Phe Ile Ser Ala Ser Lys Ser Ile Val His Pro  75
   Ser Tyr Asn Ser Asn Thr Leu Asn Asn Asp Ile Met Leu Ile Lys  90
   Leu Lys Ser Ala Ala Ser Leu Asn Ser Arg Val Ala Ser Ile Ser 105
   Leu Pro Thr Ser Cys Ala Ser Ala Gly Thr Gln Cys Leu Ile Ser 120
   Gly Trp Gly Asn Thr Lys Ser Ser Gly Thr Ser Tyr Pro Asp Val 135
   Leu Lys Cys Leu Lys Ala Pro Ile Leu Ser Asp Ser Ser Cys Lys 150
   Ser Ala Tyr Pro Gly Gln Ile Thr Ser Asn Met Phe Cys Ala Gly 165
   Tyr Leu Glu Gly Gly Lys Asp Ser Cys Gln Gly Asp Ser Gly Gly 180
   Pro Val Val Cys Ser Gly Lys Leu Gln Gly Ile Val Ser Trp Gly 195
   Ser Gly Cys Ala Gln Lys Asn Lys Pro Gly Val Tyr Thr Lys Val 210
   Cys Asn Tyr Val Ser Trp Ile Lys Gln Thr Ile Ala Ser Asn     224

### (2) INFORMATION FOR SEQ ID NO: 23

(i) SEQUENCE CHARACTERISTICS:
   (A) LENGTH: 701 base pairs
   (B) TYPE: Nucleic acid
   (C) STRANDEDNESSS: single stranded
   (D) TOPOLOGY: linear
(ii) MOLECULE TYPE: DNA
(xi) SEQUENCE DESCRIPTION: SEQ ID NO: 23
   CAT ATG GTG GAT GAT GAT GAT AAG ATC GTT GGC GGC TAC ACC TGT    45
       Met Val Asp Asp Asp Asp Lys Ile Val Gly Gly Tyr Thr Cys    14
   GGC GCC AAT ACC GTC CCG TAC CAG GTG TCC CTG AAT TCT GGC TAC    90
   Gly Ala Asn Thr Val Pro Tyr Gln Val Ser Leu Asn Ser Gly Tyr    29
   CAC TTC TGT GGT GGC TCC CTC ATC AAC TCC CAG TGG GTG GTA TCA   135
   His Phe Cys Gly Gly Ser Leu Ile Asn Ser Gln Trp Val Val Ser    44
   GCG GCC CAC TGC TAC AAG TCC GGC ATC CAG GTG CGT CTG GGC GAG   180
   Ala Ala His Cys Tyr Lys Ser Gly Ile Gln Val Arg Leu Gly Glu    59
   GAT AAC ATC AAC GTC GTG GAG GGC AAT GAG CAG TTC ATC TCC GCA   225
   Asp Asn Ile Asn Val Val Glu Gly Asn Glu Gln Phe Ile Ser Ala    74
   TCC AAG TCC ATC GTG CAC CCG TCC TAC AAC TCC AAC ACT CTG AAC   270
   Ser Lys Ser Ile Val His Pro Ser Tyr Asn Ser Asn Thr Leu Asn    89
   AAT GAC ATC ATG CTG ATC AAG CTC AAG TCC GCC GCA TCC CTG AAC   315
   Asn Asp Ile Met Leu Ile Lys Leu Lys Ser Ala Ala Ser Leu Asn   104
   TCC CGC GTG GCC TCC ATC TCT CTG CCG ACC TCC TGT GCC TCC GCC   360
   Ser Arg Val Ala Ser Ile Ser Leu Pro Thr Ser Cys Ala Ser Ala   119
   GGC ACG CAG TGC CTC ATC TCT GGC TGG GGC AAC ACT AAG AGC TCT   405
   Gly Thr Gln Cys Leu Ile Ser Gly Trp Gly Asn Thr Lys Ser Ser   134
   GGC ACC TCC TAC CCA GAC GTG CTG AAG TGC CTG AAG GCT CCT ATC   450
   Gly Thr Ser Tyr Pro Asp Val Leu Lys Cys Leu Lys Ala Pro Ile   149
   CTG AGC GAT TCC TCC TGT AAG TCC GCC TAC CCT GGC CAG ATT ACC   495
   Leu Ser Asp Ser Ser Cys Lys Ser Ala Tyr Pro Gly Gln Ile Thr   164
   AGC AAC ATG TTC TGT GCC GGC TAC CTG GAG GGC GGC AAG GAT TCC   540
   Ser Asn Met Phe Cys Ala Gly Tyr Leu Glu Gly Gly Lys Asp Ser   179
   TGT CAG GGT GAT TCT GGT GGC CCT GTG GTC TGC TCC GGC AAG CTC   585
   Cys Gln Gly Asp Ser Gly Gly Pro Val Val Cys Ser Gly Lys Leu   194
   CAA GGC ATC GTC TCC TGG GGT TCC GGC TGT GCC CAG AAG AAC AAG   630
   Gln Gly Ile Val Ser Trp Gly Ser Gly Cys Ala Gln Lys Asn Lys  209
   CCT GGC GTC TAC ACC AAG GTC TGT AAC TAT GTG TCC TGG ATT AAG  675
   Pro Gly Val Tyr Thr Lys Val Cys Asn Tyr Val Ser Trp Ile Lys  224
   CAG ACC ATA GCT TCC AAT TAGGATCC                              701
   Gln Thr Ile Ala Ser Asn                                       230

### (2) INFORMATION FOR SEQ ID NO: 24

(i) SEQUENCE CHARACTERISTICS:
   (A) LENGTH: 230 base pairs
   (B) TYPE: protein
   (C) STRANDEDNESSS: single stranded
   (D) TOPOLOGY: linear
(ii) MOLECULE TYPE: protein
(xi) SEQUENCE DESCRIPTION: SEQ ID NO: 24
   Met Val Asp Asp Asp Asp Lys Ile Val Gly Gly Tyr Thr Cys Gly   15
   Ala Asn Thr Val Pro Tyr Gln Val Ser Leu Asn Ser Gly Tyr His   30
   Phe Cys Gly Gly Ser Leu Ile Asn Ser Gln Trp Val Val Ser Ala   45
   Ala His Cys Tyr Lys Ser Gly Ile Gln Val Arg Leu Gly Glu Asp   60
   Asn Ile Asn Val Val Glu Gly Asn Glu Gln Phe Ile Ser Ala Ser   75
   Lys Ser Ile Val His Pro Ser Tyr Asn Ser Asn Thr Leu Asn Asn   90
   Asp Ile Met Leu Ile Lys Leu Lys Ser Ala Ala Ser Leu Asn Ser  105
   Arg Val Ala Ser Ile Ser Leu Pro Thr Ser Cys Ala Ser Ala Gly  120
   Thr Gln Cys Leu Ile Ser Gly Trp Gly Asn Thr Lys Ser Ser Gly  135
   Thr Ser Tyr Pro Asp Val Leu Lys Cys Leu Lys Ala Pro Ile Leu  150
   Ser Asp Ser Ser Cys Lys Ser Ala Tyr Pro Gly Gln Ile Thr Ser  165
   Asn Met Phe Cys Ala Gly Tyr Leu Glu Gly Gly Lys Asp Ser Cys  180
   Gln Gly Asp Ser Gly Gly Pro Val Val Cys Ser Gly Lys Leu Gln  195
   Gly Ile Val Ser Trp Gly Ser Gly Cys Ala Gln Lys Asn Lys Pro  210
   Gly Val Tyr Thr Lys Val Cys Asn Tyr Val Ser Trp Ile Lys Gln  225
   Thr Ile Ala Ser Asn                                          230

## Claims

1. A recombinant DNA expression vector comprising the DNA sequence of Sequence I.D. 21.

2. The vector of claim 1 that is plasmid pRMG4.

3. A recombinant DNA expression vector comprising the DNA sequence of Sequence I.D. 23.

4. The vector of claim 3 that is plasmid pRMG7.

5. A method of producing bovine trypsin comprising culturing a host cell transformed with the vector of claim 1 under conditions appropriate for production of bovine trypsin.

6. The method of claim 5 wherein said host cell is a lon- host cell.

7. A method of producing bovine trypsinogen comprising culturing a host cell transformed with the vector of claim 3 under conditions appropriate for production of bovine trypsinogen.

8. The method of claim 7 wherein said vector is plasmid pRMG7.

9. The method of claim 7 wherein said host cell is a lon- host cell.

10. A method of producing bovine trypsin comprising
(a) culturing a host cell transformed with the vector of claim 3 under conditions appropriate for production of bovine trypsinogen
(b) recovering the trypsinogen from step (a) and
(c) enzymatically converting the trypsinogen to trypsin

11. A method for converting human proinsulin to human insulin comprising treating human proinsulin with biosynthetically produced trypsin.
